# EUROPEAN PATENT SPECIFICATION

(11) **EP 2 737 038 B1**
(45) Date of publication and mention of the grant of the patent: **30.09.2015**
(21) Application number: 12740170.1
(22) Date of filing: 27.07.2012
(51) Int. Cl.: C11D 1/10, C11D 1/28, C11D 1/52, C11D 1/88, C11D 3/20, C11D 10/04, C11D 17/00, A61K 8/44, A61K 8/46, A61Q 19/10

(54) **CONCENTRATED FATTY ACYL AMIDO SURFACTANT COMPOSITIONS**
KONZENTRIERTE FETTSÄURE-ACYLAMID-TENSIDZUSAMMENSETZUNGEN
COMPOSITIONS CONCENTRÉES DE TENSIOACTIF PORTANT UN GROUPE (ACYL GRAS)AMIDO

(30) Priority: 28.07.2011 US 201161512434 P; 05.01.2012 US 201213343731
(43) Date of publication of application: 04.06.2014
(73) Proprietor: Unilever PLC, London,EC4Y 0DY (GB); Unilever N.V., 3013 AL Rotterdam (NL)
(72) Inventor: HERMANSON, Kevin, David, Trumbull, Connecticut 06611 (US); HARICHIAN, Bijan, Trumbull, Connecticut 06611 (US); VETHAMUTHU, Martin, Swanson, Trumbull, Connecticut 06611 (US)
(74) Representative: Tansley, Sally Elizabeth
(86) International application number: PCT/EP2012/064768
(87) International publication number: WO 2013/014264

(56) References cited:
- EP-A1- 1 801 194
- US-A- 5 529 712
- US-A1- 2002 028 954
- US-A1- 2005 176 615

## Description

### BACKGROUND OF THE INVENTION

### Field of the Invention

The invention concerns concentrate compositions formulated with high levels of fatty acyl amido surfactant, particularly but not exclusively, for skin cleansing products.

### The Related Art

High energy costs related to shipping have forced the reengineering of many consumer products. For instance, liquid laundry detergents traditionally have contained up to 80% water and been packaged in relatively large plastic containers. In recent years the market has favorably responded to concentrated laundry liquids. The Small and Mighty® variant of 'all' brand laundry liquid provides the same cleaning amount as the traditional full size yet with substantially less water and in a much smaller container.

Industrial chemicals similar to consumer liquid products can be shrunk by eliminating unnecessary water. Illustrative are commercially available surfactants such as sodium cocoyl glycinate shipped by producers as a 25% surfactant active in water. Shipment of water is wasteful of transportation energy.

We have been investigating concentrated surfactant cleansing compositions. Of particular interest have been more convenient ways of providing fatty acyl amido surfactants such as sodium cocoyl glycinate. A concentrate has been developed as part of the present invention wherein a polyol instead of water becomes a carrier for the surfactant. One of the problems we have encountered is that the concentrate is a pasty material. It is difficult to handle the paste both through processing equipment and delivery thereafter to a distant location. It would be highly useful were the concentrate to be rendered a solid and advantageously that could be ground into a particle or chip format.

### SUMMARY OF THE INVENTION

A solid concentrate of a C₈-C₂₂ acyl amido surfactant is provided which includes:
(i) from 35 to 90% by weight of a C₈-C₂₂ acyl amido surfactant having a structure (I): 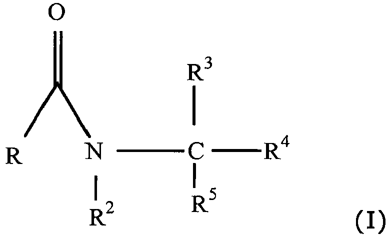 wherein R is a C₇-C₂₂ saturated or unsaturated alkyl radical; R² is hydrogen, CH₂COOX or a C₁-C₅ alkyl radical; R³ is hydrogen; R⁴ is selected from the group consisting of (CH₂)ₘCO₂X, (CH₂)ₘSO₃X, CH₂NR²(CH₂)ₘOH and glucosyl radicals; R⁵ is selected from the group consisting of hydrogen, hydroxyphenyl, C₁-C₆ hydroxyalkyl, C₁-C₁₀ alkyl, benzyl, hydroxybenzyl, alkylcarbamido, thioalkyl, and carboxylic radicals; X is selected from hydrogen, metal ions, amine salts and C₁-C₄ alkyl radicals; and m ranges from 0 to 6; and
(ii) from 10 to 60% by weight of polyol; and
(iii) from 1 to 20% by weight of C₈-C₂₂ fatty acids; and
wherein the concentrate has a pH ranging from 9 to 13.

### DETAILED DESCRIPTION OF THE INVENTION

Now we have discovered that a concentrate of a C₈-C₂₂ acyl amido surfactant in combination with a polyol and a small amount of C₈-C₂₂ fatty acids can be rendered into a solid form. Solidification is achieved by adjusting pH of the concentrate to lie between 9 and 13, preferably between 10.5 and 13, and optimally between 11 and 12.8. The pH value is measured by dissolution of 10% of the concentrate in distilled water.

By the term "solid" is meant to define a material exhibiting a Penetration Force value between 8 and 250, preferably between 10 and 200, more preferably between 15 and 175, and optimally between 25 and 160 Newton as measured by a TA.XTplus Texture Analyzer type of penetrometer.

Concentrate compositions of this invention will contain a C₈-C₂₂ acyl amido surfactant of structure (I) which is: 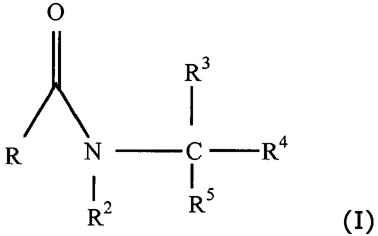 wherein R is a C₇-C₂₂ saturated or unsaturated alkyl radical; R² is hydrogen, CH₂COOX or a C₁-C₅ alkyl radical; R³ is hydrogen; R⁴ is selected from the group consisting of (CH₂)ₘCO₂ (CH₂)ₘSO₃X, CH₂NR²(CH₂)ₘOH and glucosyl radicals; R⁵ is selected from the group consisting of hydrogen, hydroxyphenyl, C₁-C₆ hydroxyalkyl, C₁-C₁₀ alkyl, benzyl, hydroxybenzyl, alkylcarbamido, thioalkyl, and carboxylic radicals; X is selected from hydrogen, metal ions, amine salts and C₁-C₄ alkyl radicals; and m ranges from 0 to 6; and
(ii) from 10 to 60% by weight of polyol; and
(iii) from 1 to 20% by weight of C₈-C₂₂ fatty acids; and
wherein the concentrate has a pH ranging from 9 to 13.

Most particularly, the surfactants of structure (I) are C₈-C₂₂ acyl amido carboxylic or sulfonic acid or salts thereof. The salts may have any type of cationic counterion X, but preferably are selected from sodium, potassium or mixed cations. The most preferred R group is a mixture of C₈-C₁₈ fatty acids with a primary chain length being C₁₂. These mixtures are often known as cocoates being derived from coconut fatty acids.

Among the preferred species of structure (I) are the sodium, potassium or ammonium salts of cocoyl glycinate, cocoyl sarcosinate, cocoyl taurate, lauroyl glycinate, lauroyl sarcosinate, lauroyl taurate, myristyl glycinate, myristyl sarcosinate, myristyl taurate, palmitoyl glycinate, palimtoyl sarcosinate, palmitoyl taurate and combinations thereof.

Amounts of the C₈-C₂₂ acyl amido surfactants of structure (I) range from 35 to 90%, preferably from 40 to 80%, and optimally from 50 to 75% by weight of the concentrate.

A polyol will also be present in the concentrate compositions. Illustrative polyols are glycerol, propylene glycol, dipropylene glycol, pentylene glycol, butylene glycol, isobutylene glycol and combinations thereof. Most preferred are glycerol and propylene glycol. Amounts of the polyol in the concentrate range from 10 to 60%, preferably from 20 to 50%, and optimally from 25 to 45% by weight.

Another material present in the concentrate is C₈-C₂₂ fatty acids. Illustrative fatty acids include lauric, myristic, palmitic, stearic, oleic, linoleic, behenic acids and combinations thereof. Amounts of the fatty acids in the concentrate range from 1 to 20%, preferably from 2 to 15%, and optimally from 4 to 10% by weight.

Advantageously, the concentrate may be substantially free of water. By substantially free of water is meant amounts from 0 to 10%, preferably from 0 to 5%, more preferably from 0 to 3%, still more preferably from 0 to 1%, and especially from 0.05 to 1% by weight of water. Water of hydration (such as found associated or complexed with any of the components) is not considered to count as part of water present in the concentrate.

Dependent upon how the concentrate is prepared, there may be present triglycerides, diglycerides, monoglycerides and mixtures thereof. Illustrative monoglycerides are monoglyceryl laurate, monoglyceryl oleate, monoglyceryl linoleate, monoglyceryl myristate, monoglyceryl stearate, monoglyceryl palmitate, monoglyceryl cocoate and mixtures thereof. Illustrative diglycerides include glyceryl dilaurate, glyceryl dioleate, glyceryl dilinoleate, glyceryl dimyristate, glyceryl distearate, glyceryl diisostearate, glyceryl dipalmitate, glyceryl cocoate, glyceryl monolaurate monomyristate, glyceryl monolaurate monopalmitate and mixtures thereof. Illustrative but non-limiting triglycerides include oils and fats such as coconut oil, corn oil, palm kernel oil, palm oil, soybean oil, cottonseed oil, rapeseed oil, canola oil, sunflowerseed oil, sesame oil, rice oil, olive oil, tallow, castor oil and mixture thereof. Amounts of the glycerides may range from about 0.05 to about 15%, preferably from about 0.1 to about 10%, more preferably from about 0.5 to about 6%, and were usually from about 1 to about 3% by weight.

Alkalinity can be introduced by a variety of alkaline materials. Illustrative but non-limiting are calcium oxide, calcium hydroxide, potassium oxide, potassium hydroxide, sodium oxide, sodium hydroxide, magnesium oxide, magnesium hydroxide, calcium phosphate, magnesium phosphate, potassium phosphate, sodium phosphate (in forms such as trisodium phosphate, disodium hydrogen phosphate), sodium carbonate, sodium bicarbonate and mixtures thereof. Amounts of alkaline materials may range from 0.01 to about 10%, preferably from about 0.05 to about 5%, more preferably from about 0.1 to about 4%, and usually from about 1 to about 4% by weight.

The term "comprising" is meant not to be limiting to any subsequently stated elements but rather to encompass non-specified elements of major or minor functional importance. In other words the listed steps, elements or options need not be exhaustive. Whenever the words "including" or "having" are used, these terms are meant to be equivalent to "comprising" as defined above.

It should be noted that in specifying any range of concentration or amount, any particular upper concentration can be associated with any particular lower concentration or amount.

The following examples will more fully illustrate the embodiments of this invention. All parts, percentages and proportions referred to herein and in the appended claims are by weight unless otherwise illustrated.

### EXAMPLE

A series of concentrates were prepared to evaluate the effect of pH on the rheology.

### Sample Preparation

Samples were prepared by first grinding sodium hydroxide pellets (98% Pure Sigma Aldrich S8045) into a fine powder. This sodium hydroxide powder was then dispersed in either glycerol or water. Calcium oxide and sodium glycine were then dispersed in the glycerol/water mixture. After dispersing the calcium oxide and sodium glycine, the glycerol mixtures were heated to 130 °C and the water mixtures were then heated to 80 °C while being mixed with an impeller blade. Once the mixture was heated to the desired temperature, sodium cocoyl glycinate (Amilite GCS-11) was mixed into the glycerol/water mixture. The sample was mixed for 30 minutes until the sodium cocoyl glycinate formed a soft paste/liquid. Coconut fatty acid was then added and the sample and mixing of the sample continued for another 20 minutes. Mixing of the sodium cocyl glycinate and fatty acid was done in an open container which allowed any excess water to evaporate. After all of the ingredients were well mixed the sample was cooled to room temperature.

### Penetration Force Procedure

Formulation hardness was measured using a standard method which is typically used to assess soap bar hardness. In this method, samples were first pressed into cylindrical pellets. The force required to push a metal cone 10 mm into each pellet was then measured using a TA.XTplus Texture Analyzer. In this method solid samples will register a higher force for penetration than pasty samples.

The pellets were made by inserting a sample into a cylindrical mold. An Instron 5567 was then used to push a cylindrical plunger into the mold in order to compress the sample into a cylindrical pellet at room temperature. The sample was compressed at a rate of 5 mm/min, and until a final force of 15 kN was achieved. The final pellet was 40 mm in diameter and 25 mm in height.

The sample hardness was measured using the TA.XTplus Texture Analyzer fitted with a 30 degree stainless steel cone. The samples were measured at room temperature with a pre-test speed of 10 mm/s, a 1 mm/s test speed and a 10 mm/s post-test speed. The samples were compressed in the texture analyzer to a target distance of 10 mm and using a trigger force of 0.049 N. Three compression measurements were taken for each sample. The compression force at a distance of 10 mm was compared for the samples. A commercial Ivory® soap bar which had also been compressed into a pellet was used as reference sample (Penetration Force of 16.1N).

The pH values were measured by dispersing 10% of a sample in a deionized water. These dispersions were then allowed to mix at room temperature for 24 hours before pH was measured. Measurements were done at room temperature with a Fisher Scientific® Accumet AP61 pH meter.

### Concentrate Compositions and Results

The compositions and resultant rheologies are reported in Table I below.

**TABLE I**

| | Sample (Weight %) | | | | | | | | | | |
|---|---|---|---|---|---|---|---|---|---|---|---|
| Component | A | B | C | D | E | F | G | H | I | J | K |
| Sodium Cocoyl Glycinate (100% Active)* | 57.2 | 57.2 | 57.2 | 57.2 | 61.9 | 57.2 | 57.2 | 57.2 | 70.0 | 63.0 | 41.9 |
| Coconut Fatty Acid** | 4.7 | 4.7 | 4.7 | 4.7 | 0.0 | 4.7 | 4.7 | 4.7 | 4.7 | 4.7 | 20.0 |
| Glycerol | 37.6 | 36.0 | 27.8 | 29.4 | 29.4 | 28.3 | 0.0 | 35.6 | 15 | 22.0 | 27.8 |
| Sodium Glycine | 0.0 | 1.6 | 1.6 | 0.0 | 0.0 | 1.6 | 1.6 | 0.0 | 1.6 | 1.6 | 1.6 |
| Water | 0.0 | 0.0 | 0.0 | 0.0 | 0.0 | 0.0 | 27.8 | 0.0 | 0.0 | 0.0 | 0.0 |
| Calcium Oxide | 0.50 | 0.50 | 0.50 | 0.50 | 0.50 | 0.00 | 0.50 | 0.50 | 0.50 | 0.50 | 0.50 |
| Sodium Hydroxide | 0.00 | 0.00 | 8.17 | 8.17 | 8.17 | 8.17 | 8.17 | 2.04 | 8.17 | 8.17 | 8.17 |
| pH | 7.2 | 7.8 | 12.2 | 12.3 | 12.4 | 12.4 | 12.3 | 11.9 | 12.6 | 12.5 | 12.4 |
| Penetration Force (Newton) | 5.3 | 2.7 | 29.8 | 32.0 | 56.8 | 19.7 | 8.4 | 15.0 | 148.7 | 154.7 | 65.1 |

| | | | | | | | | | | | |
|---|---|---|---|---|---|---|---|---|---|---|---|
| * Sourced as Amilite GCS-11. ** Sourced as Prifac 7901. | | | | | | | | | | | |

Samples A and B were formulated to achieve a pH of 7.2 and 7.8, respectively. At these levels of low basicity, the resultant concentrates were pastes exhibiting Penetration Force values of only 5.3 and 2.7, respectively. An increase of alkalinity within the range of 12.2 through 12.6 as seen in Samples C, D, E, F, H, I, J, and K resulted in solid concentrates of Penetration Force values greater than 10 Newton. Sample G contained a significant amount of water which substantially lowered the Penetration Force value.

## Claims

1. A solid concentrate of a C₈-C₂₂ acyl amido surfactant comprising:
(i) from 35 to 90% by weight of a C₈-C₂₂ acyl amido surfactant having a structure (I) as follows: 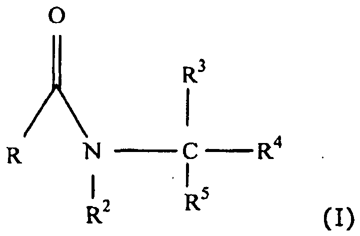 wherein R is a C₇-C₂₂ saturated or unsaturated alkyl radical; R² is hydrogen, CH₂COOX or a C₁-C₅ alkyl radical; R³ is hydrogen; R⁴ is selected from the group consisting of (CH₂)ₘCO₂X (CH₂)ₘSO₃X, CH₂NR²(CH₂)ₘOH and glucosyl radicals; R⁵ is selected from the group consisting of hydrogen, hydroxyphenyl, C₁-C₆ hydroxyalkyl, C₁-C₁₀ alkyl, benzyl, hydroxybenzyl, alkylcarbamido, thioalkyl, and carboxylic radicals; X is selected from hydrogen, metal ions, amine salts and C₁-C₄ alkyl radicals; and m ranges from 0 to 6; and
(ii) from 10 to 60% by weight of polyol; and
(iii) from 1 to 20% by weight of C₈-C₂₂ fatty acids; and
(iv) from 0 to 10% by weight of water
wherein the concentrate has a pH ranging from 9 to 13.

2. The concentrate according to claim 1 wherein the pH ranges from 11 to 12.8.

3. The concentrate according to claim 1 wherein the polyol is selected from the group consisting of glycerol, propylene glycol, dipropylene glycol, pentylene glycol, butylene glycol, isobutylene glycol and combinations thereof.

4. The concentrate according to claim 1 wherein the polyol is selected from the group consisting of glycerol, propylene glycol or combinations thereof.

5. The concentrate according to claim 1 wherein the acyl amido surfactant is selected from the group consisting of salts of cocoyl glycinate, cocoyl sarcosinate, cocoyl taurate, lauroyl glycinate, lauroyl sarcosinate, lauroyl taurate, myristyl glycinate, myristyl sarcosinate, myristyl taurate, palmitoyl glycinate, palmitoyl sarcosinate, palmitoyl taurate and combinations thereof.

6. The concentrate according to claim 1 further comprising from 0 to 1% by weight of water.

7. The concentrate according to claim 1 having a Penetration Force value between 8 and 250 Newton.

8. The concentrate according to claim 1 having a Penetration Force value between 10 and 250 Newton.

9. The concentrate according to claim 1 wherein C₈-C₂₂ acyl amido surfactant is present in an amount from 50 to 75% by weight.

## Patentansprüche

1. Festes Konzentrat eines C₈-C₂₂ Acylamidotensides, umfassend:
(i) 35 bis 90 Gew.-% eines C₈-C₂₂ Acylamidotensides mit einer Struktur (I) wie folgt: 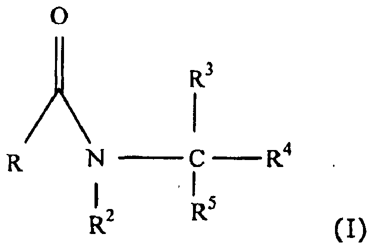 wobei R ein gesättigter oder ungesättigter C₇-C₂₂ Alkylrest ist; R² Wasserstoff, CH₂COOX oder ein C₁-C₅ Alkylrest ist; R³ Wasserstoff ist; R⁴ aus der Gruppe bestehend aus (CH₂)ₘCO₂X, (CH₂)ₘSO₃X, CH₂NR²(Ch₂)ₘOH und Glucosylresten ausgewählt ist; R⁵ aus der Gruppe bestehend aus Wasserstoff, Hydroxyphenyl-, C₁-C₆ Hydroxyalkyl-, C₁-C₁₀ Alkyl-, Benzyl-, Hydroxybenzyl-, Alkylamido-, Thioalkyl- und Carboxylresten ausgewählt ist; X aus Wasserstoff, Metallionen, Aminsalzen und C₁-C₄ Alkylresten ausgewählt ist; und m von 0 bis 6 reicht; und
(ii) 10 bis 60 Gew.-% Polyol; und
(iii) 1 bis 20 Gew.-% C₈-C₂₂ Fettsäuren; und
(iv) 0 bis 10 Gew.-% Wasser,
wobei das Konzentrat einen pH-Wert im Bereich von 9 bis 13 hat.

2. Konzentrat gemäß Anspruch 1, wobei der pH-Wert im Bereich von 11 bis 12,8 liegt.

3. Konzentrat gemäß Anspruch 1, wobei das Polyol aus der Gruppe bestehend aus Glycerin, Propylenglycol, Dipropylenglycol, Pentylenglycol, Butylenglycol, Isobutylenglycol und Kombinationen davon ausgewählt ist.

4. Konzentrat gemäß Anspruch 1, wobei das Polyol aus der Gruppe bestehend aus Glycerin, Propylenglycol oder Kombinationen davon ausgewählt ist.

5. Konzentrat gemäß Anspruch 1, wobei das Acylamidotensid aus der Gruppe bestehend aus Cocoylglycinat, Cocoylsarcosinat, Cocoyltaurat, Lauroylglycinat, Lauroylsarcosinat, Lauroyltaurat, Myristoylglycinat, Myristoylsarcosinat, Myristoyltaurat, Palmitoylglycinat, Palmitoylsarcosinat, Palmitoyltaurat und Kombinationen davon ausgewählt ist.

6. Konzentrat gemäß Anspruch 1, ferner umfassend 0 bis 1 Gew.-% Wasser.

7. Konzentrat gemäß Anspruch 1 mit einem Wert der Eindringkraft zwischen 8 und 250 Newton.

8. Konzentrat gemäß Anspruch 1 mit einem Wert der Eindringkraft zwischen 10 und 250 Newton.

9. Konzentrat gemäß Anspruch 1, wobei das C₈-C₂₂ Acylamidotensid in einer Menge von 50 bis 75 Gew.-% vorhanden ist.

## Revendications

1. Concentré solide de tensioactif acylamido en C₈-C₂₂ comprenant :
(i) de 35 à 90 % en poids d'un tensioactif acylamido en C₈-C₂₂ présentant une structure (I) comme suit : 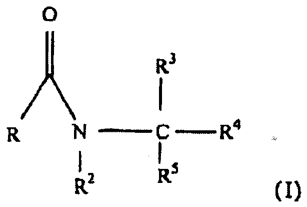 dans laquelle R est un radical alkyle saturé ou insaturé en C₇-C₂₂ ; R² est un radical hydrogène, CH₂COOX ou alkyle en C₁-C₅ ; R³ est l'hydrogène ; R⁴ est choisi dans le groupe constitué de radicaux (CH₂)ₘCO₂X, (CH₂)ₘSO₃X, CH₂NR²(CH₂)ₘOH et glucosyle ; R⁵ est choisi dans le groupe constitué de radicaux hydrogène, hydroxyphényle, hydroxyalkyle en C₁-C₆, alkyle en C₁-C₁₀, benzyle, hydroxybenzyle, alkylcarbamido, thioalkyle, et carboxyliques ; X est choisi parmi des radicaux hydrogène, ions métalliques, sels d'amines et alkyle en C₁-C₄ ; et m est compris entre 0 et 6 ; et
(ii) de 10 à 60 % en poids de polyol ; et
(iii) de 1 à 20 % en poids d'acides gras en C₈-C₂₂ ; et
(iv) de 0 à 10 % en poids d'eau
dans lequel le concentré présente un pH compris entre 9 et 13.

2. Concentré selon la revendication 1, dans lequel le pH est compris entre 11 et 12,8.

3. Concentré selon la revendication 1, dans lequel le polyol est choisi dans le groupe constitué de glycérol, propylène glycol, dipropylène glycol, pentylène glycol, butylène glycol, isobutylène glycol et de combinaisons de ceux-ci.

4. Concentré selon la revendication 1, dans lequel le polyol est choisi dans le groupe constitué de glycérol, propylène glycol ou de combinaisons de ceux-ci.

5. Concentré selon la revendication 1, dans lequel le tensioactif acylamido est choisi dans le groupe constitué de sels de glycinate de cocoyle, sarcosinate de cocoyle, taurate de cocoyle, glycinate de lauroyle, sarcosinate de lauroyle, taurate de lauroyle, glycinate de myristyle, sarcosinate de myristyle, taurate de myristyle, glycinate de palmitoyle, sarcosinate de palmitoyle, taurate de palmitoyle et de combinaisons de ceux-ci.

6. Concentré selon la revendication 1 comprenant de plus de 0 à 1 % en poids d'eau.

7. Concentré selon la revendication 1 présentant une valeur de force de pénétration entre 8 et 250 Newton.

8. Concentré selon la revendication 1 présentant une valeur de force de pénétration entre 10 et 250 Newton.

9. Concentré selon la revendication 1, dans lequel le tensioactif acylamido en C₈-C₂₂ est présent dans une quantité de 50 à 75 % en poids.
